# EUROPEAN PATENT APPLICATION

(11) **EP 4 317 450 A1**
(43) Date of publication of application: **07.02.2024**
(21) Application number: 22774286.3
(22) Date of filing: 23.03.2022
(51) Int. Cl.: C12P 21/06, A23B 4/20, C08B 37/08, A61K 38/17

(54) **CARTILAGE EXTRACT WITH EFFECT OF IMPROVING IMMUNE RESPONSE, PREPARATION METHOD THEREFOR, AND USE THEREOF**

(30) Priority: 23.03.2021 CN 202110308583
(71) Applicant: Anhui Semnl Biotechnology Co., Ltd, Huaibei, Anhui 235000 (CN)
(72) Inventor: WANG, Haiyan, Beijing 100062 (CN); LIU, Aiqing, Beijing 100062 (CN); YAN, Zheng, Beijing 100062 (CN); LIU, Lige, Beijing 100062 (CN)
(74) Representative: Impact Intellectual Property LLP
(86) International application number: PCT/CN2022/082557
(87) International publication number: WO 2022/199631

(57) **Abstract**

A cartilage extract with the effect of improving immune response, a preparation method therefor, and a use thereof. The cartilage extract uses animal cartilage as a raw material, and a cartilage extract containing an effective amount of undenatured type II collagen for improving immune response is obtained by means of adding sodium hyaluronate and lentinan at a mass ratio of 4:1 to 1:1, being capable of simultaneously preventing microbial spoilage of the cartilage extract and denaturation of the type II collagen during the storage period, and thereby improving the safety and storability of the product. The obtained cartilage extract has the advantages of high safety and effectiveness and has the effects of improving immune response, increasing the content of serum anti-inflammatory factors such as TGF-β, and reducing the content of inflammatory factors such as TNF-α, and can therefore be added to health food or functional food as a food raw material.

## Description

### Cross-referencing of related applications

This application claims priority to the Chinese patent application for an invention entitled "Cartilage extract with effect of improving immune response, preparation method therefor, and use thereof", application number 202110308583.4, the full text of which is hereby incorporated into this document by reference.

### Technical Field

The present invention relates to a cartilage extract with effect of improving immune response, preparation method therefor, and use thereof. More specifically, the present invention relates to a cartilage extract with effect of improving immune response and containing an effective amount of non-denatured type II collagen for improving immune response, and to a method of preparing said cartilage extract and its use.

### Background

Collagen is a biological macromolecule, which is the main component of animal connective tissue and the most abundant and widely distributed functional protein in mammals. Collagen generally accounts for 25-30% by mass of the total amount of protein in animals, and in some animals even up to 80 % by mass or more. There are 27 types of collagens found so far, and the common types are type I, type II, type III, type V, and type XI. Collagen has been widely used in food, health food, biomedical materials, and clinical medical use because of its low toxicity, low antigenicity, low immunity, guidance of cell regeneration, and better compatibility with the human body. Currently, most of the commercially available collagen is hydrolyzed collagen, whose main components are peptides with molecular weight distribution in the range of 200-20,000 Dalton (Da).

Cartilage, i.e. cartilage tissue, is a kind of connective tissue with mainly a supporting role, consisting of chondrocytes and cartilage matrix, rich in type II collagen and cartilage polysaccharides. type II collagen is a typical fibrous protein, consisting of three identical α-chains, the main chain is mainly β-folded and irregularly curled structure, and does not contain α-helix; glycine residues account for about 30% of the total amino acids, which is 4-5 times more than the content of hydroxyproline; it does not contain tryptophan and the content of aromatic amino acids is small; the molecular masses of hydrolyzed α1 peptide chains are all between 110-130 kilodaltons (KDa); andthe denaturation temperatures are all above 34°C.

Cartilage extracts containing non-denatured type II collagen from cartilage have been reported in the prior art. For example, a method for preparing a cartilage extract containing non-denatured type II collagen is disclosed in CN106916870A, wherein said method sequentially includes the steps of defatting, sterilization, homogenization, enzymatic digestion, filtration, and drying. In the Examples of CN106916870A, the prepared cartilage extract contains 3.9 to 12.6 % by mass of type II collagen. However, the above CN106916870A is silent on how the cartilage extract prepared therein achieves stable storage.

For protein products including type II collagen, it is very important to achieve protein preservation (preventing the growth and reproduction of spoilage bacteria and pathogenic bacteria) during the storage period. Currently available means to achieve effective preservation during storage include high-temperature treatment (H), low-temperature refrigeration or freezing (t), acidification (pH), reduction of redox value, the addition of preservatives, and so on. However, the above-mentioned high-temperature treatment (H), low-temperature refrigeration or freezing (t), acidification (pH), and reduction of redox value will almost always result in protein denaturation while achieving preservative, and the addition of preservatives does not meet the modem consumer trend of pursuing green and preservative-free. In addition, with the growth of storage time, the risk of protein denaturation will gradually increase.

In order to effectively prevent protein denaturation while achieving preservation during the storage period of the protein, some methods have been proposed in the prior art, one of which teaches the use of a combination of an anti-microbial medium (e.g., hypochlorite) and an ionized salt (e.g., sodium chloride, potassium chloride) to improve preservation of a protein product (e.g., collagen type II) and prevent protein denaturation during the storage process. However, the anti-microbial medium (e.g., hypochlorite) have oxidizing properties and are inherently harmful to human health, leading to potential health risks if ingested into the human body, while the ionized salts (e.g., sodium chloride, potassium chloride) may lead to high levels of sodium and potassium in the body, with the risk of triggering hypernatremia or hyperkalemia, if ingested in excessive quantities. Therefore, this method is not ideal.

For type II collagen molecules, the immune response must be elicited by intact undenatured type II collagen molecules according to the immune response mechanism, the growth of spoilage and/or pathogenic bacteria during storage of type II collagen poses a significant risk of use due to product safety issues. On the other hand, once type II collagen is denatured during storage, it is unable to provide an effective immune response. Therefore, there has been a need in the prior art for techniques to prevent protein denaturation while preserving type II collagen-containing products.

### Summary of the Invention

The present invention is made to solve the above-mentioned problems in the prior art. It is an object of the present invention to provide a cartilage extract with effect of improving immune response, said cartilage extract contains an effective amount of type II collagen for improving immune response, and is capable of effectively preventing denaturation of said type II collagen during storage while preserving it, thereby maintaining the improved immune response after storage. The present invention also provides preparation methods and uses of said cartilage extract.

Surprisingly, the present inventors have found that by adding a specific mass ratio of sodium hyaluronate and lentinan during the preparation of said cartilage extract containing non-denatured type II collagen, said sodium hyaluronate and lentinan are able to simultaneously achieve a preservative effect and prevent denaturation of said type II collagen during the storage of the prepared cartilage extract. The present invention is based on this discovery.

Specifically, one embodiment of the present invention relates to a cartilage extract with effect of improving immune response, the cartilage extract containing an effective amount of non-denatured type II collagen for improving the immune response, wherein the cartilage extract is supplemented with sodium hyaluronate and lentinan, wherein the mass ratio of said sodium hyaluronate to lentinan is 4:1 to 1:1.

In a preferred embodiment, said cartilage extract contains from 2 to 20% by mass of non-denatured type II collagen.

In a preferred embodiment, the mass ratio of said sodium hyaluronate to lentinan is 4:1-2:1.

In a preferred embodiment, said cartilage extract is made from cartilage as raw material and the total amount of the added sodium hyaluronate and lentinan is from 10 to 60% by mass, preferably from 20 to 30% by mass, based on the total mass of cartilage used as raw material.

Another embodiment of the present invention relates to a method of preparing cartilage extract with effect of improving immune response, wherein during the preparation of cartilage extract from cartilage, sodium hyaluronate and lentinan are added, wherein the mass ratio of said sodium hyaluronate to lentinan is 4:1-1:1.

In a preferred embodiment, said method comprises the following steps:
1) using cartilage as raw material and removing the lipid component from the cartilage;
2) homogenizing by pulverizing the cartilage obtained in step 1) from which the lipid component has been removed;
3) adding to the homogenate obtained in step 2) at least one selected from elastase, fig protease, and ginger protease for enzymatic digestion;
4) adding sodium hyaluronate and lentinan to the enzymatic digest obtained in step 3);
5) freeze-drying the material obtain in step 4) to obtain the cartilage extract.

In a preferred embodiment, in said step 3), the trio of elastase, fig protease, and ginger protease are added.

In a preferred embodiment, the mass ratio of said sodium hyaluronate to lentinan is 4:1-2:1.

In a preferred embodiment, the total amount of the added sodium hyaluronate and lentinan is from 10 to 60% by mass, preferably from 20 to 30% by mass, based on the total mass of cartilage which is used as raw material.

In a preferred embodiment, the cartilage extract after freeze-drying in said step 5) is further pulverized to obtain a pulverized material and then stored at room temperature.

In a preferred embodiment, a further step of disinfection with ethanol is included between said step 1) and step 2).

In a preferred embodiment, a step of filtering and collecting the filtrate is also included between steps 3) and 4).

Another embodiment of the present invention relates to the use of the above-mentioned cartilage extracts with effect of improving immune response in the preparation of a formulation for improving immune response.

In a preferred embodiment, said formulation has an effective storage period of not less than 36 months at room temperature.

Technical effect: The cartilage extract of the present invention avoids the addition of traditional preservative substances such as hypochlorite and traditional substances preventing protein denaturation such as sodium chloride and potassium chloride, thus avoiding the risks to human health associated with the intake of these traditional preservative substances and/or substances preventing protein denaturation. As an alternative, sodium hyaluronate and lentinan added to the cartilage extract of the present invention are low-calorie beneficial substances and are beneficial for improving human immunity, and therefore are not only harmless but also beneficial to human health. Moreover, the addition of sodium hyaluronate and lentinan to the cartilage extract of the present invention can effectively avoid the denaturation of type II collagen while achieving preservative effects.

As a result, the cartilage extract of the present invention, when prepared as a formulation for improving immune response, can maintain an effective effect of improving immune response even after being stored at room temperature for not less than 36 months, thereby protecting the human body from tissue damage caused by immune response overreactivity.

### Brief Description of the Drawings

Figure 1 shows transmission electron microscopy image of non-denatured type II collagen obtained according to Example 1.
Figure 2 shows the results of western blotting of non-denatured type II collagen obtained according to Example 1, where the right lane is the type II collagen obtained in Example 1.
Figure 3 illustrates the ameliorative effect of cartilage extract on sodium iodoacetate (MIA)-induced immune response overreactivity as verified in Experimental Example 1.
Figure 4 illustrates the ameliorative effect of cartilage extract on sodium iodoacetate (MIA)-induced immune response overreactivity as verified in Experimental Example 2.

### Detailed Description of the Invention

The present invention is described in detail hereinafter in connection with specific embodiments. However, it will be apparent to those skilled in the art that the following specific embodiments are for illustrative purposes only and should not be construed as limiting the scope of the present invention.

In the present specification, the term "non-denatured" means that the type II collagen is not denatured and has an intact triple helix structure.

In the present specification, the term "about" is used to indicate ±5% of the specified value when used to limit a value.

In the present specification, the term "%" indicates % by mass if not otherwise specified.

In the present specification, the term "room temperature" indicates the ambient temperature, i.e., without special warming or cooling treatment.

In the present specification, the term "an effective amount of ••••••for improving immune response" means that the amount of non-denatured type II collagen in the cartilage extract is effective for improving immune response and that the amount can be determined and configured according to actual needs.

A first embodiment of the present invention relates to a cartilage extract with effect of improving immune response, the cartilage extract contains an effective amount of non-denatured type II collagen for improving the immune response, and the cartilage extract is supplemented with sodium hyaluronate and lentinan, wherein the mass ratio of said sodium hyaluronate to lentinan is from 4:1 to 1:1.

In the first embodiment described above, said cartilage extract may preferably contain from 2 to 20% by mass of non-denatured type II collagen.

In the above-mentioned first embodiment, the amount of the added sodium hyaluronate and lentinan can be reasonably determined according to practical needs. Preferably, the total amount of the added sodium hyaluronate and lentinan is 10 to 60% by mass, more preferably 20 to 30% by mass, based on the total mass of cartilage which is used as raw material.

A second embodiment of the present invention relates to a method for preparing a cartilage extract with effect of improving immune response as described above, said method comprises the addition of sodium hyaluronate and lentinan during the preparation of a cartilage extract from cartilage, wherein the mass ratio of said sodium hyaluronate to lentinan is 4:1 to 1:1.

In the second embodiment described above, said method preferably comprises the following steps:
1) using cartilage as raw material and removing the lipid component from the cartilage;
2) homogenizing by pulverizing the cartilage obtained in step 1) from which the lipid component has been removed;
3) adding to the homogenate obtained in step 2) at least one select from elastase, fig protease, and ginger protease for enzymatic digestion:
4) adding sodium hyaluronate and lentinan to the enzymatic digest obtained in step 3);
5) freeze-drying the material obtained in step 4) to obtain the cartilage extract.

In the second embodiment described above, there is no particular limitation on the source of cartilage used as raw material, which can, for example, be derived from the cartilage of fish, cattle, pigs, sheep, chicken, duck, etc. Thus, the raw material used in the present invention has the advantage of being from a wide range of sources and being simple and easy to obtain. Accordingly, the cartilage extract prepared in the present invention can be added as a food ingredient to ordinary food, health food, and pharmaceutical product because it is made from the cartilage of common domestic animals, poultry, and other animals as raw materials, which is a safe source.

In the second embodiment described above, said cartilage may be pre-treated before defatting. For example, said cartilage may be diced using a dicing machine after removing non-cartilaginous components such as meat, fascia, redbone, etc. The size of said dice is not particularly limited and may for example be 4 × 4 mm.

In the second embodiment described above, the defatting of the cartilage can be carried out in a manner known in the art, using a defatting agent commonly used in the art, without any particular limitation. Said defatting agent may be, for example, Na₂CO₃, NaHCO₃, Na₂SiO₃, Na₃PO₄ solution, etc., preferably Na₂CO₃, NaHCO₃ solution. The concentration of said defatting agent may be in the range of 0.005 to 0.1% by mass, preferably in the range of about 0.01 to 0.04% by mass, most preferably about 0.02% by mass. The time for defatting is not particularly limited and can be, for example, from 3 to 10 hours, preferably from 5 to 7 hours, most preferably about 6 hours. After defatting and draining, it can be washed with purified water to a pH of 6 to 7, preferably to a pH of about 6.5.

In the second embodiment described above, a disinfection treatment may also be carried out after defatting the cartilage and before homogenization. Said disinfection treatment can be carried out using, for example, ethanol at a concentration of about 75%. There is no particular limitation on the number of times and duration of the disinfection, for example, 2 to 5, preferably 3 to 4 disinfections can be carried out, and each disinfection can be carried out, for example, for about 1 to 3 hours, preferably about 2 hours. After the disinfection is complete, a wash with a purified water can be carried out to remove the ethanol. The disinfection and purified water washing can be performed at a pH of 6.0 to 7.5, preferably at a pH of 6.5 to 7.0.

In the second embodiment described above, in step 2), said homogenization may be carried out using a homogenizer. Said homogenizer is not particularly limited and can be, for example, a colloid mill. As an example, said homogenization process can be carried out by grinding the defatted cartilage to a size of 100 to 200 mesh.

In the second embodiment described above, in step 3), at least one selected from elastase, fig protease, and ginger protease is added for enzymatic digestion. The type, amount, and enzymatic activity of these proteases can be selected according to the actual needs, as long as the denaturation of type II collagen is minimized and the degradation of other proteins is increased. Preferably, at least one selected from elastase, fig protease, and ginger protease may be added in an amount of 0.5 to 1.5% by mass, more preferably about 0.75 to 1.0% by mass, based on the total mass of said cartilage as raw material. Preferably, all three of elastase, fig protease, and ginger protease are added for enzymatic digestion, and the elastase, fig protease, and ginger protease may be added in an amount of 0.1 to 1% by mass, 0.1 to 0.5% by mass, and 0.1 to 0.5% by mass, respectively, based on the total mass of said cartilage as raw material. The pH conditions during said enzymatic digestion can be suitably determined according to the actual situation, for example, the enzymatic digestion can be carried out at a pH of 6 to 8, preferably a pH of about 7.5. The temperature and duration of the enzymatic digestion may also be determined as appropriate to the circumstances, for example, from 5 to 50 hours at room temperature, preferably from 10 to 30 hours, and more preferably from 20 to 27 hours.

In the second embodiment described above, between said step 3) and step 4), a filtration step may also be included for removing large particulate matter from the enzymatic digest and collecting the filtrate. Said filtration may be carried out at room temperature by using a sieve, for example, a 1 to 10-mesh, preferably a 5-mesh sieve.

In the second embodiment described above, in said step 4), sodium hyaluronate and lentinan are added to the enzymatic digest.

The amount of the added sodium hyaluronate and lentinan can be reasonably determined according to the actual needs. However, from the point of view of obtaining the best preservative effect and prevention of type II collagen denaturation, the total amount of the added sodium hyaluronate and lentinan may preferably be from 10 to 60% by mass, preferably from 20 to 30% by mass, based on the total mass of said cartilage as raw material, and the preservative effect and prevention of type II collagen denaturation can be optimized.by adding this amount of the sodium hyaluronate and lentinan.

In the second embodiment described above, the freeze-drying in said step 5) may preferably be carried out under vacuum. Said freeze-drying process preferably comprises a pre-cooling treatment and a subsequent drying process. Said pre-cooling treatment may be carried out at a temperature of, for example, not higher than -40°C, and the final temperature of the said drying process may be controlled at a temperature of, for example, 10°C to 50°C, for example at about 30°C. By means of this freeze-drying treatment, the cartilage extract is obtained.

In the above second embodiment, preferably after said step 5), said cartilage extract can also be pulverized to obtain a pulverized material. Said pulverization is preferably carried out at low temperatures, said low temperatures may for example be below 10° C, preferably below 6° C. Said pulverization may be carried out, for example, by superfine pulverization, the size of the pulverizedmaterial may be suitably determined according to actual needs, for example, controlled at 100-300 mesh, preferably controlled at 150-250 mesh. The pulverized material can be effectively stored at room temperature for not less than 36 months.

A third embodiment of the present invention relates to the use of the above-mentioned cartilage extract with effect of improving immune response in the preparation of aformulation for improving immune response.

In the third embodiment described above, said formulation has an effective storage period of not less than 36 months at room temperature.

Herein, there is no particular limitation on the form of the formulation in said third embodiment, for example said formulation may be a dosage form for gastrointestinal administration such as a commonly used bulk, tablet, granule, capsule, emulsion, suspension, etc., or said formulation may be a non-gastrointestinal dosage form such as an injection, ointment, hard paste, paste, patch, etc.

Alternatively, said formulation in said third embodiment can be administered at a dose of, for example, 1 to 60 mg/kg body weight/day, more preferably at 2 to 30 mg/kg body weight/day, further preferably at 4 to 15 mg/kg body weight/day. In particular, the formulation in said third embodiment has a positive therapeutic effect for rheumatoid arthritis, in particular at relatively high doses (e.g., above 8 mg/kg/day).

The cartilage extract prepared according to the invention contains an effective amount of non-denatured type II collagen for improving the immune response, and the addition of sodium hyaluronate and lentinan makes it possible to preserve and prevent denaturation of said type II collagen even after a long storage period (not less than 36 months), i.e., it remains effective in improving the immune response even after a long period of storage. The cartilage extract prepared according to the present invention is safe without toxic side effects, and thus can be further used in the preparation of pharmaceuticals, health foods, and food products.

### Examples

The present invention is described in further detail by way of the following examples. However, the following examples are for exemplary purposes only and should not be construed as limiting the scope of the present invention. If not otherwise specified, the reagents used in the following embodiments are of analytically pure grade.

### Example 1 Preparation of cartilage extract

(1) Pretreatment: 3 kg of chicken breast cartilage was selected, thawed and the chicken meat, fascia, and red bone were removed from the cartilage, and then cut into 4×4 mm dices using a dicing machine;
(2) Defatting: The cartilage is defatted at room temperature for 6 h using 7 kg of NaHCO₃ solution with a final concentration of 0.02% by mass. The cartilage was washed with purified water to pH 6.5 after being defatted and drained;
(3) Disinfection: Disinfection is started by adding a concentration of 75 % ethanol by mass for 4 times, 2 hours each time, and after disinfection is finished, a wash is performed with purified water until there is no ethanol odor, and the pH is controlled at 6.5 to 7.0;
(4) Homogenization: 6 kg of purified water is added to the cartilage dices treated in step (3) and the cartilage is ground to 100-200 mesh using a colloid mill;
(5) Enzymatic digestion: The pH of the slurry obtained from step (4) is adjusted to 7.5, 15 g of elastase, 5 g of fig protease and 5 g of ginger protease are added, and enzymatic digestion is carried out at a temperature of 25°C for 24 hours;
(6) Filtration: A filtration is performed using a 5-mesh sieve to remove large particulate material from the solution and collect the filtrate;
(7) Preservation: 500 g of sodium hyaluronate and 500 g of lentinan with Aw 0.73 were added;
(8) Drying: The material obtained from step (7) was vacuum freeze-dried with the pre-cooling temperature not higher than -40°C and the final temperature of the drying process controlled at 30°C. The cartilage extract was obtained after freeze-drying with an Aw of 0.23.
(9) Pulverization: The freeze-dried cartilage extract was superfinely pulverized below 4°C, and the mesh size of the pulverized material was controlled at 200 mesh to obtain a freeze-dried product with a shelf life of 3 years at room temperature.

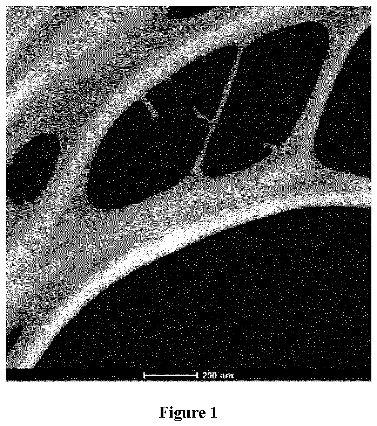

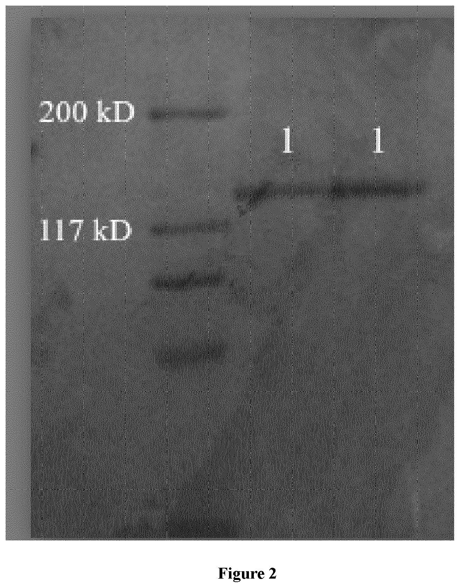

The content of non-denatured type II collagen was 19.5% by ELISA method (the supplier of the kit was Shanghai Jianglai Biotechnology Co., Ltd.). The morphology of non-denatured type II collagen was detected by transmission electron microscopy, and the result was shown in Figure 1. The western blot was used to detect type II collagen, and the result was shown in the right lane of Figure 2.

### Experimental Example 1: Effect of cartilage extract on TNF-α in sodium iodoacetate (MIA)-stimulated cartilage tissue of SD rats

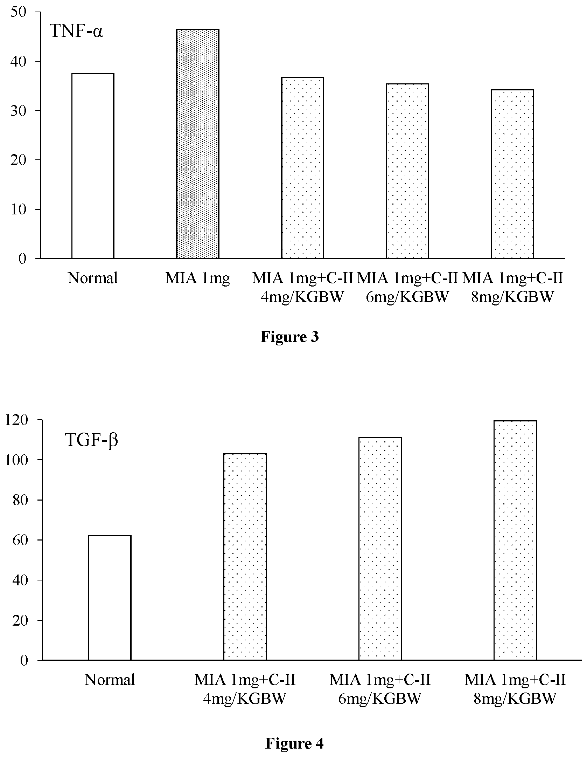

SD (Sprague-Dawley) male rats, weighing 200 g~230 g, SPF (Specific Pathogen Free) grade, were purchased from Liaoning Changsheng Biotechnology Co., Ltd. Except for the blank control group (normal group), all rats were injected with 25 µL (40 mg/mL) of sodium iodoacetate (MIA) solution prepared in saline, i.e., 1 mg MIA per rat, into the knee joint cavity, and the blank control group (normal group) was injected with the same volume of saline. Cartilage extract prepared from Example 1 (low, medium, and high dose groups, respectively) was added to the feed in an amount of 4, 6, or 8 mg/kg body weight (kgBW)/day starting on the day of molding. The normal group was given standard feed, and after 5 consecutive weeks, blood was collected from the abdominal aorta, centrifuged at 3000 rpm for 10 min to obtain serum, and the content of TNF-α (produced by Shanghai Enzyme Link Biotechnology Co., Ltd.) in rat serum was determined by ELISA. The experimental results are shown in Figure 3. The low, medium and high dose groups of cartilage extract could significantly reduce the TNF-α content in serum, which were significantly different from that of the model group (P<0.01, P<0.05).

The above results demonstrate that the cartilage extract prepared in Example 1 has a reducing effect on the level of TNF-α in the serum in a dose-dependent manner (i.e., it inhibits the production of TNF-α, which is associated with inflammatory or autoimmune diseases). This directly suggests that the cartilage extract prepared in Example 1 inhibits immune response overreactivity, thereby helping to return the immune response to normal levels.

### Experimental Example 2: Effect of cartilage extract on TGF-β content in MIA-induced SD rats

SD male rats, weighing 200 g~230 g, SPF grade, were purchased from Liaoning Changsheng Biotechnology Co. All rats except the blank control group (normal group) were injected with 25 µL (40 mg/mL) of sodium iodoacetate (MIA) solution prepared in saline, i.e., 1 mg MIA/each, into the knee joint cavity, and the blank control group (normal group) was injected with the same volume of saline. The cartilage extract prepared from Example 1 was added to the feed in an amount of 4, 6, or 8 mg/kgBW/day (low, medium, and high dose groups, respectively) starting on the day of molding. The normal group was given standard feed, and after 5 consecutive weeks, blood was collected from the abdominal aorta, and centrifuged at 3000 rpm for 10 minutes to obtain serum, and of the content of TGF-β (produced by Shanghai Enzyme Link Biotechnology Co., Ltd.) in rat serum was determined by ELISA. The experimental results are shown in Figure 4. The low, medium and high dose groups of cartilage extract could significantly increase the TGF-β content in rat serum, which were significantly different from the model group (P<0.05, P<0.01).

The above results demonstrate that the cartilage extract prepared in Example 1 has an increasing effect on the serum TGF-β content in a dose-dependent manner (i.e., it induces the production of TGF-β, which is associated with alleviating the inflammatory response). This directly indicates that the cartilage extract prepared in Example 1 has an inhibitory effect on immune response overreactivity, thus helping to return the immune response to normal levels.

### Experimental Example 3: Effect of different polysaccharide substances in preserving and preventing type II collagen denaturation in cartilage extracts

Erythritol, chitosan, sodium hyaluronate, and lentinan were selected in the amounts of experiments 1-9 in the following table, respectively, and mixed with cartilage extract obtained from 4 kg of cartilage treated according to steps (1) to (6) in Example 1, and tested for Aw, as well as for colony count, molds, and yeasts after 6 months of accelerated experiments. In experiments 10-11 in Table 1 below, the polysaccharide substances were replaced by equal weights of NaCl and KCl, respectively.

**Table 1 Aw and microorganisms of different polysaccharide substances mixed with cartilage extracts**

| Testing items | Experiment 1 | Experiment 2 | Experiment 3 | Experiment 4 | Experiment 5 | Experiment 6 | Experiment 7 | Experiment 8 | Experiment 9 | Experiment 10 | Experiment 11 |
|---|---|---|---|---|---|---|---|---|---|---|---|
| | Erythritol (1000g) | Chitosan ( 1000g) | Sodium hyaluronate (1000g) | lentinan ( 1000g) | Sodium hyaluronate (850g) + lentinan (150g) | Sodium hyaluronate (800g) + lentinan (200g) | Sodium hyaluronate (700g) + lentinan (300g) | Sodium hyaluronate (600g) + lentinan (400g) | Sodium hyaluronate (500g) + lentinan (500g | Sodium chloride ( 1000g) | Potassium chloride ( 1000g) |
| Aw before drying | 0.71 | 0.69 | 0.74 | 0.72 | 0.70 | 0.73 | 0.74 | 0.71 | 0.69 | 0.73 | 0.71 |
| Aw after drying | 0.23 | 0.19 | 0.32 | 0.29 | 0.25 | 0.29 | 0.26 | 0.25 | 0.27 | 0.29 | 0.30 |
| Non-denaturing type II collagen content (%) | 18.8% | 18.3% | 19.7% | 18.7% | 19.5% | 20.1 % | 20.6% | 20.7% | 20.5% | 18.6% | 19.3% |
| Total number of bacterial colonies (cfti/g) | 100 | <10 | 90 | 150 | 130 | 60 | 100 | 90 | 30 | 50 | 120 |
| Molds and yeasts (cfu/g) | <10 | <10 | <10 | <10 | <10 | <10 | <10 | <10 | <10 | <10 | <10 |
| After 6 months of accelerated experimentation Aw | 0.56 | 0.45 | 0.54 | 0.62 | 0.47 | 0.25 | 0.26 | 0.27 | 0.27 | 0.23 | 0.26 |
| Non-denatured type II collagen content after 6 months of accelerated experiment (%) | 12.3% | 11.5% | 9.1% | 12.3% | 14.1 % | 20.4% | 20.5% | 20.6% | 20.3% | 18.8% | 19.0% |
| Total number of bacterial colonies after 6 months of accelerated experiment (cfti/g) | 440 | 520 | 390 | 560 | 450 | 60 | 100 | 130 | 80 | 50 | 120 |
| molds and yeasts after 6 months of accelerated experiment (cfti/g) | 10 | 20 | 10 | 10 | 10 | <10 | <10 | <10 | <10 | <10 | <10 |

The above Table 1 clearly shows that Aw became high in the accelerated experiments in experiments 1-5, leading to microbial growth and a decrease in non-denatured type II collagen content. On the other hand, in experiments 10-11, sodium chloride and potassium chloride as used in the prior art were used, respectively, which could maintain the Aw substantially unchanged, inhibit microbial growth, and maintain the non-denatured type II collagen content, but the use of high sodium and high potassium was detrimental to human health. In Experiments 6-9, on one hand, Aw can be maintained substantially unchanged, the microbial growth can be inhibited, and the non-denaturing type II collagen content can be maintained, and on the other hand, the use of sodium hyaluronate and lentinan did not have adverse effects on human health.

The present invention is specifically described above in connection with the embodiments, but it will be apparent to those skilled in the art that various amendments may be made thereto without departing from the subject matter and scope of the present invention. Such amendments are within the scope of the invention of the present application.

## Claims

1. A cartilage extract with effect of improvingimmune response, said cartilage extract containing an effective amount of non-denatured type II collagen for improving immune response, **characterized in that** the cartilage extract is supplemented with sodium hyaluronate and lentinan, wherein the mass ratio of sodium hyaluronate to lentinan is 4:1-1:1.

2. The cartilage extract with effect of improvingimmune response according to claim 1, **characterized in that** said cartilage extract contains from 2 to 20% by mass of non-denatured type II collagen.

3. The cartilage extract with effect of improvingimmune response according to claim 1 or 2, **characterized in that** the mass ration of said sodium hyaluronate to lentinan is 4:1-2:1.

4. The cartilage extract with effect of improvingimmune response according to any one of claims 1 to 3, **characterized in that** said cartilage extract is made from cartilage as raw material, wherein the total amount of the added sodium hyaluronate and lentinan is from 10 to 60% by mass, preferably 20 to 30% by mass, based on the total mass of cartilage which is used as raw material.

5. A method for preparing the cartilage extract with effect of improving immune response according to claim 1, **characterized in that** the method comprises adding sodium hyaluronate and lentinan during the preparation process of the cartilage extract from cartilage, wherein the mass ratio of sodium hyaluronate to lentinan is 4:1-1:1.

6. The method according to claim 5, **characterized in that** said method comprises the following steps:
1) using cartilage as raw material and removing the lipid component from the cartilage;
2) homogenizing by pulverizing the cartilage obtained in step 1) from which the lipid component has been removed;
3) adding to the homogenate obtained in step 2) at least one selected from elastase, fig protease, and ginger protease for enzymatic digestion;
4) adding sodium hyaluronate and lentinan to the enzymatic digest obtained in step 3);
5) freeze-drying the material obtained in step 4) to obtain cartilage extract.

7. The method according to claim 6, **characterized in that** in step 3), the triad of elastase, fig protease, and ginger protease is added.

8. The method according to any one of claims 5 to 7, **characterized in that** the mass ratio of said sodium hyaluronate to lentinan is 4:1-2:1.

9. The method according to any one of claims 5 to 8, **characterized in that** the total amount of the added sodium hyaluronate and lentinan is from 10 to 60% by mass, preferably from 20 to 30% by mass, based on the total mass of cartilage which is used as raw material.

10. The method according to any one of claims 5 to 9, **characterized in that** the freeze-dried cartilage extract from said step 5) is further pulverized to obtain a pulverized material, which is then stored at room temperature.

11. The method according to any one of claims 5 to 10, **characterized in that**, between said step 1) and step 2), a further step of disinfection with ethanol is included.

12. The method according to any one of claims 5 to 11, **characterized in that** between said step 3) and step 4), a further step of filtering and collecting the filtrate is included.

13. Use of the cartilage extract with effect of improving immune response according to any one of claims 1-4 in the preparation of a formulation for improving immune response.

14. The use according to claim 13, **characterized in that** said formulation has an effective storage period of not less than 36 months at room temperature.
